# EUROPEAN PATENT APPLICATION

(11) **EP 1 908 838 A2**
(43) Date of publication of application: **09.04.2008**
(21) Application number: 06764380.9
(22) Date of filing: 15.06.2006
(51) Int. Cl.: C12N 15/29

(54) **ISOLATION AND CHARACTERISATION OF THE CSB3-1 MUTANT OF ARABIDOPSIS THALIANA AND USE THEREOF AS A REGULATOR OF RESISTANCE IN PLANTS TO DISEASES CAUSED BY BIOTROPHIC PATHOGENS**

(30) Priority: 16.06.2005 ES 200501563
(71) Applicant: Consejo Superior de Investigaciones Cientificas, 28006 Madrid (ES); Universidad Polit Cnica de Valencia, E-46022 Valencia (ES)
(72) Inventor: Gil Morrio, María J., Inst. de Biología Molecular, 46022 Valencia (ES); Coego Gonzalez, Berto, Inst. de Biología Molecular, 46022 Valencia (ES); Vera Vera, Pablo, Inst. de Biología Molecular, 46022 Valencia (ES)
(74) Representative: Ungria Lopez, Javier
(86) International application number: PCT/ES2006/070081
(87) International publication number: WO 2006/134198

(57) **Abstract**

The invention relates to the technical field of plant biotechnology and, more specifically, to the identification and characterization of the csb3-1 mutant of *Arabidopsis thaliana,* as well as to the use thereof in the regulation of resistance to diseases caused by biotrophic pathogens and to the applications of same in the generation of plants that are resistant to said type of pathogens.

## Description

The present invention belongs in the field of crop biotechnology, and most particularly it refers to the identification and characterization of the *Arabidopsis thaliana* csb3-1 mutant, as well as the role it plays in the regulation of resistance to diseases produced by biotrophic pathogens, and its applications in the generation of plants resistant to these types of pathogens.

### STATE OF THE ART

Plants react to microbial attack producing a series of inducible defenses that are integrated in a complex signaling system. In the last decade, considerable progress has been achieved in terms of understanding the molecular mechanisms plants use to defend themselves from microbial attack. This progress has been achieved by cloning, and by characterizing the resistance factors in plants that recognize the presence of pathogen avirulence factors that trigger the so called hypersensitive response (HR) (Dangl, J.L., and Jones, J.D. (2001)). Plant pathogens and integrated defence responses to infection. Nature 411, 826-833). This hypersensitive response or HR may confine the invading pathogen and thus prevent the dissemination of the infection (Agrios, G.N. (1988). Plant Pathology. Academic Press, London, U.K). In most cases, the onset of the HR causes the activation of the systemic acquired resistance (SAR) (Ross, 25 A.F. (1961). Systemic acquired resistance induced by localized virus infections in plants. Virology 14, 340-358), a defensive response mechanism that provides long term protection for the whole plant against a broad spectrum of pathogens (Durrant, W.E., and Dong, X. (2004). Systemic acquired resistance. Ann. Rev. Phytopathol. 42, 185-209). Salicylic acid (SA) is an essential signal molecule that plays a central role in SAR induction (Delaney, T. P., Uknes, S., Vernooij, B., Friedrich, L., Weymann, K., Negrotto, D., Gaffney, T., Gut-Rella, M., Kessmann, H., Ward, E., and Ryals, J. (1994). A central role of salicylic acid in plant disease resistance. Science 266, 1247-1250). Numerous mutant plants involved in the accumulation or perception of SA show an increase in their susceptibility to biotrophic pathogens, while mutant plants that produce an excess of SA show increased resistance.

In particular, several mutants of *Arabidopsis thaliana* have been isolated that show an incapability of activating the SA-dependent defensive responses (Durrant and Dong, 2004). The eds16/sid2 mutants (enhanced disease susceptibility 16 / salicylic acid induction deficiency 2) and eds5/sid1 (Rogers, E.E., and Ausubel, F.M. (1997). Arabidopsis enhanced disease susceptibility mutants exhibit enhanced susceptibility to several bacterial pathogens and alterations in PR-1 gene expression. Plant Cell 9, 305- 316.; Nawrath, C., and Metraux, J.P. (1999). Salicylic acid induction-deficient mutants of Arabidopsis express PR-2 and PR-5 and accumulate high levels of camalexin after pathogen inoculation. Plant Cell 11, 1393-1404; Wildermuth, M.C., Dewdney, J., Wu, G., and Ausubel, F.M. (2001). Isochorismate synthase is required to synthesize salicylic acid for plant defence. Nature 414, 562-565.) do not accumulate SA after being inoculated with virulent and avirulent pathogens. SID2 codes for an alleged isochorismate synthase located in the chloroplast (Wildermuth et al., 2001), while EDS5 codes for a protein associated to the membrane and located in the chloroplast that shows homology with MATE transporters (Multidrug And Toxin Extrusion) (Nawrath et al., 2002). These findings show the existence of a SA synthesis path that is located in the chloroplast and revealed the importance of this organelle in critical mechanisms of mediation and defensive response control of plants (Shah, J. (2003). The salicylic acid loop in plant defense. Curr. Opin. Plant Biol 6, 365-371). In fact, mutations in several of the genes that code for proteins located in the chloroplast result in defective SA signaling (Kachroo, P., Shanklin, J., Shah, J., Whittle, E.J., and Klessig, D.F. (2001). A fatty acid desaturase modulates the activation of defense signaling pathways in plants. Proc. Natl. Acad. Sci. U S A 98, 9448-9453.; De Leon, I.P., Sanz, A., Hamberg, M., and Castresana, C. (2002). Involvement of the Arabidopsis alpha-DOX1 fatty acid dioxygenase in protection against oxidative stress and cell death. Plant J. 29, 61-62.; Slaymaker, D.H., Navarre, D.A., Clark, D., del Pozo, 0., Martin, G.B., and Klessig, D.F. (2002). The tobacco salicylic acid-binding protein 3 (SABP3) is the chloroplast carbonic anhydrase, which exhibits antioxidant activity and plays a role in the hypersensitive defense response. Proc Natl Acad Sci U S A 99, 11640-11645.; Ishikawa, A., Okamoto, H., Iwasaki, AND., and Asahi, T. (2001). A deficiency of coproporphyrinogen III oxidase causes lesion formation in Arabidopsis. Plant J. 27, 89-99.; Mach, J.M., Castillo, A.R., Hoogstraten, R., and Greenberg, J.T. (2001). The Arabidopsis-accelerated cell death gene ACD2 encodes red chlorophyll catabolite reductase and suppresses the spread of disease symptoms. Proc. Natl. Acad. Sci. U S A 98, 771-776).

Also, a signal derived from the chloroplast to control the SA path and the defensive response activated by pathogens that is light dependent or requires the presence of a functional photosynthetic chloroplast has been involved (Genoud, T., Buchala, A. J., Chua, N-H. and Métraux, J-P. (2002). Phytochrome signalling modulates the SA-perceptive pathway in Arabidopsis. Plant J. 31, 87-95). In addition, the eds1 and pad4 mutants (phytoalexin deficient 4), block biosynthesis of salicylic acid triggered by infection with virulent pathogens (Feys, B.J., Moisan, L.J., Newman, M.A., and Parker, J.E. (2001). Direct interaction between the Arabidopsis disease resistance signaling proteins, EDS1 and PAD4. EMBO J. 20, 5400-5411.; Jirage, D., Tootle, T.L., Reuber, T.L., Frost, L.N., Feys, B.J., Parker, J.E., Ausubel, F.M., and Glazebrook, J.(1999). Arabidopsis thaliana PAD4 encodes a lipase-like gene that is important for salicylic acid signaling. Proc. Natl. Acad. Sci. USA 96, 13583-13588.) and also the one that has also been observed to occur in different disease resistant mutants (Jirage, D., Zhou, N., Cooper, B., Clarke, J.D., Dong, X., and Glazebrook, J. (2001). Constitutive salicylic acid-dependent signaling in cpr1 and cpr6 mutants requires PAD4. Plant J. 26, 395-407.; Clarke, J.D., Aarts, N., Feys, B.J., Dong, X., and Parker, J.E. (2001). Constitutive disease resistance requires EDS1 in the Arabidopsis mutants cpr1 and cpr6 and is partially EDS1-dependent in cpr5. Plant J. 26, 409-420). EDS1 and PAD4 are required to achieve the resistance conferred by a subset of R genes (that is, TIR-NB-LRR). Both proteins share homology with lipases and both act after the 5' end of SID2 and EDS5 in the regulation of SA synthesis (Glazebrook, J., Rogers, E.E., and Ausubel, F.M. (1997). Use of Arabidopsis for genetic dissection of plant defense responses. Annu. Rev. Genet. 31, 547-569.; Jirage et al., 1999; Feys et al., 2001; Nawrath, C., Heck, S., Parinthawong, N., and Métraux, J-P. (2002). EDS5, an Essential Component of Salicylic Acid-Dependent Signaling for Disease Resistance in Arabidopsis, Is a Member of the MATE Transporter Family. Plant Cell 14, 275-286). The ankyrin repeat protein NPR1 (that it does not express PR-1) was initially identified in *Arabidopsis* through a genetic selection of mutants involved in SAR (Cao, H., Glazebrook, J., Clarke, J.D., Volko, S., and Dong, X., (1997). The Arabidopsis NPR1 gene that controls systemic acquired resistance encodes a novel protein containing ankyrin repeats. Cell 88, 57-63). NPR-1 is an essential SAR regulator (Dong, X. (2004) NPR1, all things considered. Current Opinion in Plant Biology, 7, 547-552.) and also a regulator of the SAR independent defense path, ISR (Induced Systemic Response); Pieterse, C.M., and Van Loon, L.C. (2004). NPR1: the spider in the web of induced resistance signaling pathways. Curr. Opin. Plant Biol. 7, 456-464). With the SA treatment and the redox unbalancing of the cognate, NPR1 is translocated to the nucleus, where it acts as modulator of the expression of the PR gene through a selective interaction with members of the TGA subclase belonging to the zipper family of leukines transcription factors (Dong, 2004). In addition to npr1, other mutants involved in SAR have been identified. A non-exhaustive list includes dir1 (defective in induced resistance 1); Maldonado, A.M., Doerner, P., Dixon, R.A., Lamb, C.J., and Cameron, R.K. (2002). A putative lipid transfer protein involved in systemic resistance signalling in Arabidopsis. Nature 419, 399-403.), that carries a mutation in a gene that codes a protein having a similarity of sequencing with lipid transfer proteins and points in the direction of a SAR mediated signal based in mobile lipids. Also, the dth9 mutant (detachment 9) differs from npr1 in that the PR expression is not altered. The fact that treatment with SA does not re-establish resistance in dth9 plants susceptible to the disease places dth9 behind the 3' end of SA in a pathway that is parallel to that of NPR1. In addition, various genetic selections have been implemented in the search for npr1 suppressors that allow identifying novel components in the SAR signaling route, such as SNI1, SNC1, SON1 or SSI2 amongst others (Kachroo et al., 2001; Li, X., Clarke, J.D., Zhang, AND., and Dong, X. (2001). Activation of an EDS1-mediated R-gene pathway in the snc1 mutant leads to constitutive, NPR1 independent pathogen resistance. Mol. Plant Microb Interact. 14, 1131 - 1139; Li, X., Zhang, Y., Clarke, J.D., Li, AND., and Dong, X. (1999). Identification and cloning of a negative regulator of systemic acquired resistance, SNI1, through a screen for suppressors of npr1-1. Cell 98, 329- 339.; Kim, H.S., an Delaney, T.P. (2002). Arabidopsis SON1 is an F-box protein that regulates a novel induced defence response independent of both salicylic acid and systemic acquired resistance. Plant Cell 14,1469-1482.; Shah, J., Kachroo, P., and Klessig, D.F. (1999). The Arabidopsis ssi1 mutation restores pathogenesis-related gene expression in npr1 plants and renders defensive gene expression salicylic acid dependent. Plant Cell 11, 191-206.; Nandi, A., Welti, R., and Shah, J. (2004). The Arabidopsis thaliana dihydroxyacetone phosphate reductase gene suppressor of fatty acid desaturase deficiency is required for glycerolipid metabolism and for the activation of systemic acquired resistance. Plant Cell 16, 465-477.; Zbang, AND., Goritschnig, S., Dong, X., and Li, X. (2003). A gain-of-function mutation in a plant disease resistance gene leads to constitutive activation of downstream signal transduction pathways in suppressor of npr1 -1, constitutive 1. Plant Cell 15, 2636-2646). Curiously snc1 mutants (suppressor of npr1-1, constitutive 1), sni1 (suppressor of npr1 inducible1) and ssi2 (suppressor of SA insensitivity 2) show a constitutive PR expression and an increase in the biotrophic pathogen resistance. On the opposite situation, the son1 mutant (suppressor of nim1-1) contributes SAR without induction from the PR gene, and defines a new phenomenon that has been named SAR Independent Resistance or ISR (Hammond-Kosack, K.E., and Parker, J.E. (2003). Deciphering plant-pathogen communication: fresh perspectives for molecular resistance breeding. Curr. Opin. Biotechnol. 14, 177-193).

### DESCRIPTION OF THE INVENTION

One of the objects of the present invention is the isolation and characterization of a CSB3 gene mutant (SEC. ID.NO. 2) of *Arabidopsis thaliana,* known as csb3-1 (a derivative of constitutive subtilisin 3) (SEC. ID. NO.1) that increases the resistance to biotrophic pathogens and deregulated resistance in the expression of a genic promoter previously characterized that controls the expression of a pathogen-induced subtilisin protease (P69C). CSB3 was isolated by means of map-based cloning and it was shown that it codes for a 1-hydroxy-2-methyl-2-butenyl-4-diphosphate synthase, an enzyme that controls one of the terminal stages of isopentenyl diphospate (IPP) through the 4-phosphate of 2-C-methyl-D-erythrytol pathway (MEP) that occurs in the chloroplasts of higher plants. After epistasis analysis of other characterized mutants related to the defence mechanism, together with the isolation and characterization of extragenic suppressors of the csb3 mutant and the pharmacological complementation of the phenotype of csb3 plants, it is proposed that CSB3 controls the critical aspects of the SA mediated disease resistance pathway against biotrophic pathogens.

Another object of the present invention entails using the csb3-1 mutant allele in crop species of agricultural interest to obtain varieties resistant to diseases caused by biotrophic pathogens by inhibiting the CSB3 gene or the activity of the protein coded by said gene in order to increase synthesis and accumulation of salicylic acid and therefore the resistance to biotrophic pathogens.

### Isolation of the csb3 mutant

The P69C gene codes a protease similar to subtilisin, and originally identified in tomato plants (Jorda, L., and Vera, P. (2000). Local and systemic induction of two defense related subtilisin-like protease promoters in transgenic Arabidopsis plants. Luciferin induction of PR gene expression. Plant Physiol. 124, 1049-1058). Transgenic *Arabidopsis* plants that contain a region of 2,5 kb of promoter 5' of P69C merged to the ®-glucuronidase (GUS) indicator gene reveals a rapid transcriptional activation of this gene through SA and during the course of interactions, compatible and incompatible, with *Pseudomonas syringae pv. Tomato* DC3000 (Pst DC3000) (Jordá et al., 2000). Induced expression is observed in local inoculated leaves (that is, after inoculation with avrRpm1 carrying Pst DC3000, as well as in non-inoculated distal leaves of the same plant. Curiously, induced expression in inoculated leaves occurs at a distance from the area where HR is implemented. The expression of the P69C-GUS transgene occurs at points distributed along the entire length of the leafs laminae of both the local and the distal leaves, as visualized by depositing blue staining derived from the X-glue used as substrate. This expression pattern is different from that observed in other SA regulated genes, such as the PR-1 gene, which induced expression is extremely delimited and concentric to the tissues that surround the HR lesion (Bowling, S.A., Guo, A., Cao, H., Gordon, S., Kessig, D.F., and Dong, X. (1994). A mutation in Arabidopsis that leads to constitutive expression of systemic acquired resistance. Plant Cell 6, 1845 - 1857).

In order to identify the signals and mechanisms involved in the pathway leading to a distinct activation of the P69C promoter and to study the impact this pathway may have on the resistance to disease, the investigation was geared to searching for mutants using transgenic *Arabidopsis* plants containing the P69C-GUS genic construct.

The idea was that by selecting mutants showing expression of the indicator gene in healthy non-inoculated plants, the mutations that affect the defensive response could be identified. To this effect, one of the homozygotic transgenic lines containing one sole insertion of P69C-GUS was subjected to mutagenesis with ethyl methanesulphate (EMS), and then approximately 12800 M2 seeds were selected for individuals with high constitutive GUS activity. These mutants were called csb (constitutive subtilisin). A mutant with a high expression of GUS - known as csb3 - was selected. This mutant is described in detail in the present invention.

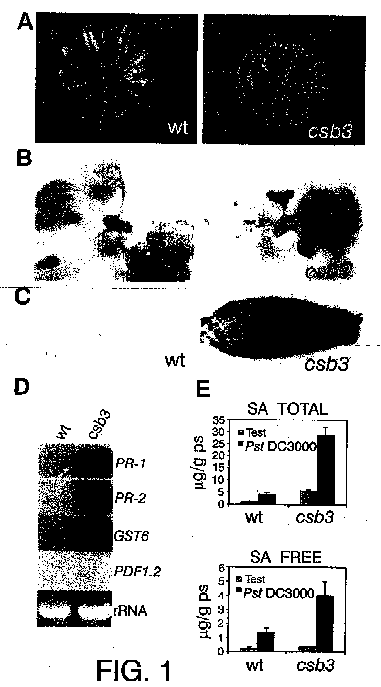

Csb3 plants may be distinguished from wild plants because of their characteristic atrophied phenotype and their curly leaves (figure 1A). A histochemical stain was done to determine GUS activity, and thus research the pattern of constitutive expression of the indicator gene of csb3 plants. As shown in figures 1B-C, no GUS activity was detected in the original wild seedlings, and the opposite was true of the csb3 seedlings, that showed GUS stain in the cotyledons and a more pronounced expression of GUS activity in the rosette leaves of plants that achieved complete growth.

Because local SA application induces the expression of the P69C-GUS in wild *Arabidopsis* plants (Jordá et al., 2000), a hypothesis was formulated postulating that SA accumulation could be increased in csb3 plants. The first step was to establish the expression of several SA inducible genes (PR-1, PR-2 and GST6) in csb3 plants. Northern type analyses shown in Figure 1D revealed that the expression of the selected genes were, in all cases, increased in intact csb3 plants but not in wild plants, pointing then in the direction of a constitutive activation of the SA dependent signaling in the csb3 mutant. On the other hand, the expression of the jasmonate/ethylene gene marker, PDF1.2, was not altered in csb3 plants (figure 1 D).

The fact that SA regulated gene expression is activated in intact csb3 plants suggested an alteration in the accumulation of SA in csb3 plants, and therefore, the levels of free SA and of conjugated salicylate glucoside (SAG) were examined in the leaf tissue of csb3 plants and of wild plants (Figure 1 E). In non-inoculated leaves of csb3 plants an increase of 8 times the SAG contents was observed when compared to that of wild plants. No significant differences were found in the concentrations of free SA, however, in the tissue of infected leaves, three days post inoculation (d.p.i.) with Pst DC3000, the amount of free SA increased eleven times in csb3 plants, while it only increased three times in wild plants.

Similar proportional increases were observed in the amount of SAG found in wild plants compared to that found in csb3 plants after inoculation (Figure 1 E). This finding suggests that the csb3 mutation not only confers an increase in SA accumulation in at rest conditions, but it also contributes to increasing the processes of synthesis and accumulation of this molecule after infection.

To analyze the inheritance pattern of the csb3 mutation, csb3/csb3 plants were backcrossed with wild CSB3/CSB3 plants containing the P69C-GUS transgene and the resulting offspring F1 and F2 was analyzed to determine the GUS stain. Because the constitutive GUS expression was absent in all F1 plants analyzed, it was concluded that the csb3 mediated constitutive GUS expression is inherited as a recessive trait. In F2 plants the expression was present in 67 of the 240 plants analyzed (the proportion being then 3:1 or non-expressing: expressing in a constitutive manner; ₂ = 0.4, 0.1 > P > 0.5), which supports the conclusion that the csb3 is a recessive mutation and also indicates that there is only one locus responsible for the csb3 phenotype.

### The csb3 mutant has increased resistance to biotrophic pathogens but not to necrotrophic pathogens

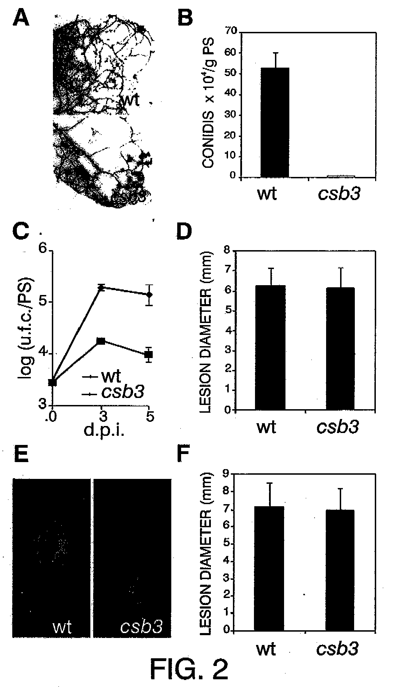

In order to determine whether there is a relationship between the signaling pathway that mediates the activation of P69C-GUS and the pathway that mediates resistance to disease, the response of the csb3 plants and of wild plants to different pathogens that cause disease in *Arabidopsis* was measured. The response of the csb3 plants to the obligatory biotrophic oomycete *Hyaloperonospora parasitica* compared to the response of wild plants is shown in Figures 2A-B. 25 csb3 plants and 25 wild plants were inoculated with the NOCO (10⁵ conidiospores/ml) *H. parastica* isolate, virulent to *Arabidopsis* Col-0. The pathogen growth was studied by direct observation of the stained hyfas present in infected leaves (Figure 2A) and by counting the conidia produced on the infected leaves (Figure 2B). Both measurements revealed a significant difference in pathogen growth in wild versus csb3 plants. While the wild plants suffered strong oomycete colonization and allowed them to develop their complete life cycle (sexual oospores and asexual conidia formation in the conidiophores), csb3 plants inhibited the growth of this pathogen drastically. This indicates that resistance to this particular biotrophic pathogen was drastically increased or susceptibility was blocked as a result of the mutation at the CSB3 locus.

Wild plants and csb3 plants were also exposed to the virulent bacterial pathogen *Pseudomonas syringae pv. tomato* DC3000 (Pst DC3000), another biotrophic pathogen of *Arabidopsis.* After inoculation the speed of growth of the pathogen was controlled in the infected leaves. The resulting growth curves are shown in Figure 2C. The number of PST DC3000 colony forming units detected in csb3 plants was 30 to 60 times lower than the number detected in wild plants after 3 or 5 days of growth, and therefore, csb3 plants also show increased resistance to this bacterial pathogen.

Susceptibility of the csb3 plants to pathogens was further analyzed using *Plectospharella cucumerina,* a necrotrophic pathogen of *Arabidopsis.* The infection of wild plants by *P. cucumerina* leads to a strong degradation of the leaf tissue that manifests as extensive lesions that increase diameter as the infection progresses along the inoculated leaf. No differences were observed in the extension of the lesions of csb3 plants versus those of wild plants (Figure 2D). To determine whether the csb3 mutation could provoke changes in susceptibility to a different necrotrophic pathogen, csb3 plants and wild plants were inoculated with *Botrytis cinerea.* The disease was scored between 5 and 10 days after inoculation by monitoring the extension of necrosis and death in inoculated leaves. Both wild plants and csb3 plants were extremely susceptible to *Botrytis,* and all plants showed necrosis accompanied by extensive proliferation of fungal mycelia (Figures 2E and 2F). In view of these results the csb3 mutation seemed not to affect plant susceptibility to necrotrophic pathogens. Based on the phenotype of the recessive csb3 mutant, the hypothesis that CSB3 could regulate negatively certain aspects of the defensive response to biotrophic pathogens could be formulated.

### Response of double csb3 mutants to Pst DC3000

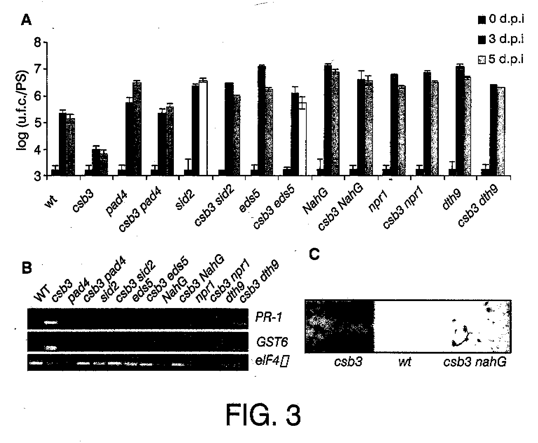

To provide additional insights about csb3 activated signaling mechanisms, a series of double mutants was generated using csb3 and other mutants in which the SA related pathway was affected. The resistance response to Pst DC3000 was examined for all these mutants (Figure 3).

The pad4 mutation compromises SA accumulation triggered by infection caused by virulent *P. syringae* (Feys et al., 2001; Jirage et al., 1999) and therefore suppresses the SA accumulation levels reached by other mutants that are resistant to biotrophic pathogens (Jirage et al., 2001). As shown in Figures 3A and 3B, both the increase in resistance to Pst DC3000 and the SA induced constitutive expression of the PR-1 gene conferred by the csb3 mutation are suppressed in doubly mutant csb3 pad4 plants. These data show that PAD4 is fully required for the signaling mechanisms in csb3 plants.

The sid2 and eds5 mutants of *Arabidopsis* show alterations in the SA synthesis and accumulation processes that occur in the chloroplast through chorismate and isochorismate pathways and both mutant plants show increased susceptibility to pathogens (Nawrath and Métraux, 1999; Wildermuth et al., 2001). In csb3 sid2 plants, as well as in csb3 eds5 plants, csb3 conferred resistance to Pst DC3000 in isolation is notably reduced (Figure 3A), and therefore, the constitutive expression of the PR-1 marker gene observed in csb3 plants was blocked at csb3 sid2 and to a lesser degree was also blocked in csb3 eds5 (Figures 3B). In consequence, increased resistance of csb3 plants depends on the SA synthesis process that takes place in the chloroplast through the action of SID2 and EDS5.

To additionally broaden our understanding of the role of SA in the increase of disease resistance of csb3 plants, the NahG transgene was subjected to introgression in csb3 plants. NahG codes a salicylate hydroxylase that blocks SA accumulation in the cytosol by degrading SA to catechol (Delaney, T.P., Uknes, S., Vernooij, B., Friedrich, L., Weymann, K., Negrotto, D., Gaffney, T., Gut-Rella, M., Kessmann, H., Ward, E., and Ryals, J. (1994). A central role of salicylic acid in plant disease resistance. 25 Science 266, 1247-1250). In csb3 NahG plants the increased resistance conferred by the csb3 mutation is again suppressed and the hypersusceptibility of NahG to Pst DC3000 (Figure 3A) is imposed. In a similar manner, the constitutive expression of the PR-1 gene disappears when the NahG transgene is present (Figure 3B).

The npr1 mutant was identified because of its increased susceptibility to biotrophic pathogens and its incapability to organize a SAR response or to express PR-1 in the response to SA treatment (Cao et al., 1997). It has been shown that NPR1 plays a key role in the mediation of many defensive responses (Dong, 2004). Curiously, csb3 npr1 plants continued to be as susceptible to Pst DC3000 as the npr1 plants (figure 3A), and their expression of the PR-1 gene was also inhibited (Figure 3B).

This indicates that the csb3 plants SA mediated resistance requires a functional NPR1 protein. Other SAR compromised mutant is the dth9 mutant plant, however, unlike the npr1 plants, dth9 plants have preserved their capacity to express PR genes in response to SA or to pathogen attacks (Mayda, E., Mauch-Mani, B., and Vera, P. (2000). Arabidopsis dth9 mutation identifies a gene involved in regulating disease susceptibility without affecting salicylic acid-dependent responses. Plant Cell 12, 2119-2128). Again, doubly mutant plants csb3 dth9 showed increased susceptibility to Pst DC3000 that can be attributed to the dth9 mutation (Figure 3A), but unlike npr1 the expression of the PR-1 marker gene that can be attributed to the csb3 mutation is kept intact (Figure 3B), indicating that csb3 resistance is also DTH9 dependent.

The constitutive expression of GUS activity, directed by the P69C promoter as observed in csb3 plants is also suppressed in all the double mutants generated, except in the csb3 dth9 plants, and it is exemplified in Figure 3C for csb3 NahG plants. Another interesting mutant analyzed by epistasis was cpr1. Cpr1 plants are more resistant to Pst DC3000, they have high levels of SA, show constitutive expression of the PR-1 gene, and also show a tendency to abnormal growth (Bowling et al., 1994), and therefore, cpr1 shares with csb3 several disease resistance aspects and growth size. Curiously, when the cpr1 mutation was subjected to introgression in the remaining csb3 background, the resulting double csb3 cpr1 mutant plants showed additive effects that resulted in an extremely dwarf phenotype. Homozygotic double mutant plants never reached the reproductive phase, and therefore propagation had to occur in the heterozygotic state. Therefore, the phenotype of the csb3 cpr1 mutant plant could suggest that the csb3 mutation and the cpr1 mutation contribute to their characteristic increase in SA accumulation and their respective phenotype of resistance increase via independent pathways after the 3' end of SA accumulation.

All these observations reinforce the insight that both SA synthesis and perception are essential mechanisms in the disease resistance trait associated to csb3.

### Isolation of csb3 suppressors

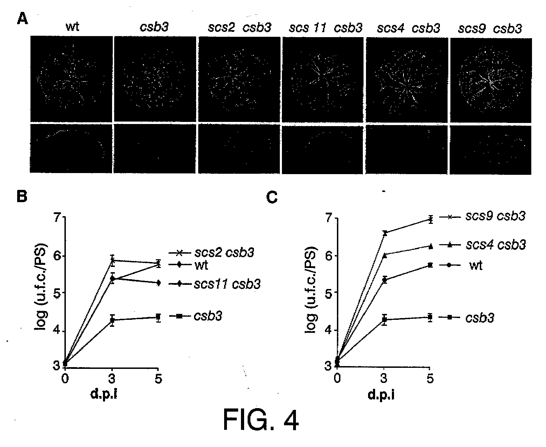

To additionally elucidate the functional role of csb3 in the defensive response of the plant, a search for csb3 suppressors was carried out by selecting mutants lacking the increase to disease resistance conferred by the csb3 mutation. Identifying suppressors of the csb3 phenotype with the corresponding reversal in the resistance response would indicate that the role of CSB3 in the participation of SA mediated resistance is a mechanism under strict genetic control. With this consideration in mind, the homozygotic csb3 seeds were again subjected to mutagenesis with EMS and approximately 10000 M2 seeds were grown and selected to identify individuals without increased resistance to Pst DC3000 attributable to the csb3 mutation. This selection process allowed isolating four scs mutants (csb3 suppressors) (more specifically scs2, scs4, scs9 and scs11; Figure 4). The four identified scs csb3 double mutants have lost the dwarf morphological phenotype that differentiates them from csb3 and are similar to wild plants in terms of architecture and the growth habit of the plant (Figure 4A), and therefore, the constitutive expression of the P69C-GUS gene (Figure 4A) and of the PR-1 gene, both attributable to the csb3 mutation were suppressed in the scs csb3 double mutants. Also, the comparative speed of growth record of Pst DC3000 in infected leaves of wild plants, of csb3 plants and of scs csb3 plants revealed that scs csb3 mutants have lost the increased resistance to Pst DC3000 characteristic of the original csb3 mutant plant (figures 4B-C). In this case, Pst DC3000 resistance reached in scs 2 csb3 and scs 11 csb3 plants is similar to that observed in wild plants, supporting a bacterial titre growth that is 8 to 40 times greater than that observed in csb3 plants (Figure 4B). The loss of increased resistance conferred by the csb3 mutation is even more noticeable in scs4 cb3 and scs9 csb3 plants, that become more susceptible to Pst DC3000 than wild plants and allow increases of bacterial growth 100 times greater when compared to csb3 plants (Figure 4C). The hypersusceptibility of scs9 csb3 plants to Pst DC3000 was even greater to that observed in npr1 plants and comparable to that recorded in dth9 plants during a side by side experiment, however, none of the scs mutations are allelic in relation to npr1, dth9 or any of the mutant plants previously mentioned. Also, genetic analysis show that the four scs mutation are not mutually allelic, they are extragenic to csb3, and the phenotype conferred by each of the scs mutations is maintained even in the absence of the csb3 mutation.

In summary, these results reinforce the hypothesis that CSB3 is an important component of the SA mediated disease resistance pathway in *Arabidopsis* plants, and its role in this pathway is additionally controlled by the SCS genic products.

### CSB3 map-based cloning

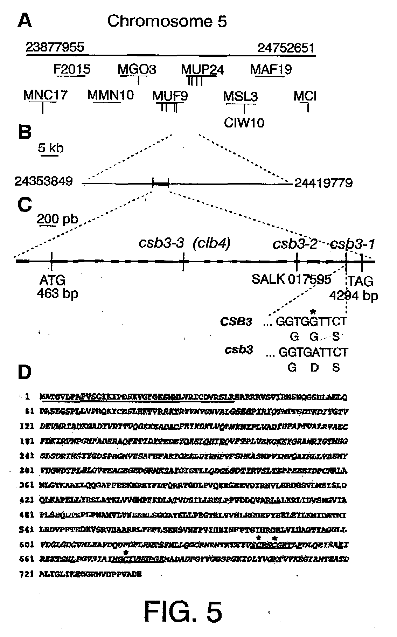

To define the CSB3 position in the chromosome map, the csb3 mutant that is in a Col-0 background was crossed with wild Landsberg *erecta* (*Ler*) plants. Homozygotic csb3 mutant plants were identified amongst the F2 offspring by their characteristic phenotypic morphology. The F2 mapping population selected to determine the PCR based molecular marker segregation was examined. For the initial mapping 38 F2 plants were tested with single sequence length polymorphism markers (SSLPS) for each of the five chromosomes (Bell, C.J., and Ecker, J.R. (1994). Assignment of 30 microsatellite loci to the linkage map of Arabidopsis. Genomics 19, 137-144). The CIW10 marker in chromosome 5 showed cosegregation with the csb3 phenotype. Additional analysis of this region showed the mutation to be localized between the nga129 and the MBK-5 markers, which are separated by 15 cM. To delimit the region where CSB3 was localized the Cereon Genomics large insertion / deletion database was used to design SSLP markers. After selecting 748 F2 plants, CSB3 was limited to two BAC (MUF9 and MUP24) clones that occupy a 66 kb region containing 21 open reading frames (ORF) (Figure 5A). The complete region was enlarged for each of these genes from csb3 plants and the PCR product sequences were determined. The sequence corresponding to the At5g60600 gene was identified as the only one that showed one sole specific mutation. This gene contains 20 exons and the csb3 mutation (from now on referred as csb3-1) represented a transition from G to A in the coding chain of exon 19, that leads to a change of aminoacids from Gly to Asp. Figures 5A-C provide an overview of the mapping strategy and the genic structure.

CSB3 is a single copy gene that codes for 1-hydroxy-2-methyl-2-butenyl-4-diphosphate [HMBPP] synthase (HDS) located in the chloroplast that catalyzes the formation of HMBPP from 2,4-cyclodiphosphate of 2-C-methyl-D-erythrytol (MEcPP) through a mechanism that is still unknown (Hecht, S., Eisenreich, W., Adam, P., Amslinger, S., Kis, K., Backer, A., Arigoni, D., and Rohdich, F. (2001). Studies on the nonmevalonate pathway to terpenes: the role of the GcpE (IspG) protein. Proc. Natl. Acad. Sci. USA 98, 14837-14842.; Kollas, A.-K., Duin, E. C., Eberl, M., Altincicek, B., Hintz, M., Reichenberg, A., Henschker, D., Henne, A., Steinbrecher, J., Ostrovsky, D. N., et al. (2002). Functional characterization of GcpE, an essential enzyme of the nonmevalonate pathway of isoprenoid biosynthesis. FEBS Lett. 532, 432-436).

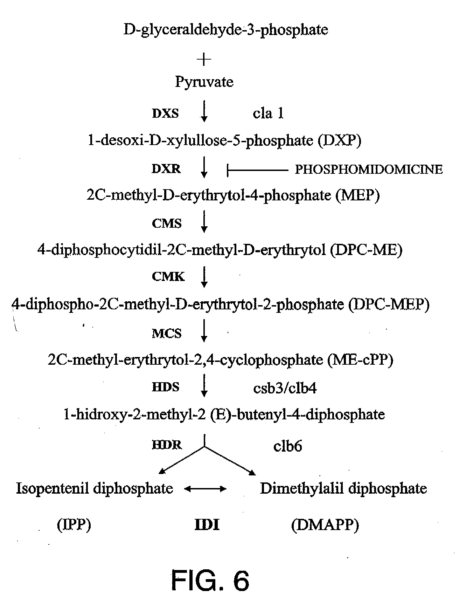

The HDS enzyme controls one of the terminal stages of the isopentenyle diphosphate (IPP) and dimethylallyl diphosphate (DMAPP) through the 4-phosphate pathway of 2-C-methyl-D- erythrytol (MEP) independently from the mevalonate produced in the chloroplast of higher plants (Figure 6). The MEP pathway localized in the plastid, together with the cytosolic mevalonate (MVA) pathway are in charge of the isopentenyle diphosphate (IPP) synthesis and the synthesis of its dimethylallyl diphosphate isomer (DMAPP), the immediate precursors for isoprenoid biosynthesis (Figure 6) (Rohmer, M. (1999). The discovery of a mevalonate-independent pathway for isoprenoid biosynthesis in bacteria, algae and higher plants. Nat. Prod. Rep. 16, 565-574). The HDS enzyme is kept in microorganisms known as GCPE (Kollas et al., 2002), in spite of the difference between the HDS enzyme of mature plants that contain a large internal protein domain that is absent in its bacterial homologue (Figure 5D). This additional domain may confer different regulatory or catalytic functions to the plant's enzyme (Querol J, Campos N, Imperial S, Boronat A, Rodríguez-Concepción M (2002)). Functional analysis of the Arabidopsis thaliana GCPE protein involved in plastid isoprenoid biosynthesis. FEBS Lett. 514: 343-346). Plant and microbial enzymes show an extremely conservative aminoacid regions near the terminal C-end, where a set of conventional Cys residue are located and participate in cluster assemblage [4Fe-4S] (aminoacids 640 to 684, Figure 5D). Identification of different mutations of *Escherichia coli* located inside or near the 4Fe-4S motif indicate that this is a critical region for enzymatic function (Sauret-Güeto S, Ramos-Valdivia A, Ibanez E, Boronat A, Rodriguez Concepcion M. (2003) Identification of lethal mutations in Escherichia coli genes encoding enzymes of the methylerythrytolphosphate pathway. Biochem Biophys Res Commun. 307, 408-415). Because the identified csb3-1 mutation is near this motif (Figure 5D), it could be argued that the mutation introduces a structural alteration that may destabilize the folding and also the correct functioning of the Fe-S cluster.

A T-DNA insertion of *Arabidopsis* (SALK 017595) was identified in the CSB3 gene, known as csb3-2 for the purposes of the present invention (Figure 5C). This mutation alters CSB3 at the 16^{th} exon level. Curiously, plants that were heterozygotic for this T-DNA insertion segregated albino seedlings that were mortal. Also, in a search for mutants with compromised chloroplast development, it was identified very recently (Gutiérrez-Nava, M.L., Gillmor, C.S., Jiménez, L.F., Guevara-Garcia, A., and León, P. (2004). CHLOROPLAST BIOGENESIS genes act cell and noncell autonomously in early chloroplast development. Plant Physiol. 135, 471-482.) that the clb4 mutant (chloroplast biogenesis) carried an EMS induced mutation at the same locus in which the change of transition results in the appearance of a premature termination codon at exon 9 (Figure 5C). To lend uniformity, this last allele was called, for the purpose of the present invention, csb3-3/clb4. In a manner similar to csb3-2, albino seedlings possessing the csb3-3/clb4 mutation were also mortal (Gutierrez-Nava et al., 2004). It is interesting to observe here that other T-DNA mutants that alter the ORF of other genes in the MEP pathway (Budziszewski, G.J., et al. (2001). Arabidopsis genes essential for seedling viability: Isolation of insertional mutants and molecular cloning. Genetics 159, 1765-1778) produce seedlings with the same mortal albino phenotype. This suggests that the MEP pathway is essential for the plant and that the csb3-1 allele identified in the present invention represents a partial loss of function and not a null mutant.

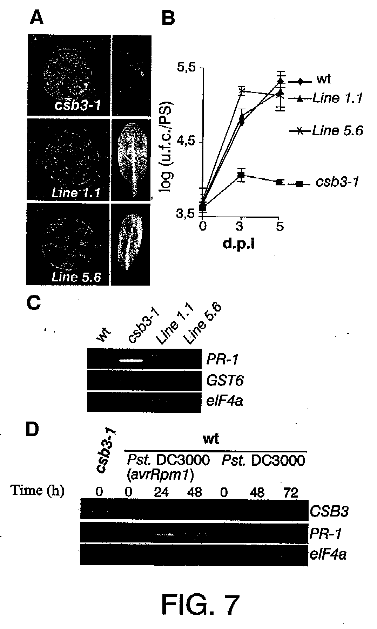

To confirm that At5g60600 corresponds to the CSB3 gene the *Agrobacterium tumefaciens* mediated transformation was used to introduce in the csb3-1 plants a cDNA that would correspond to At5g60600 under the control of the 35S promoter of CaMV (35S-CSB3 construct). Several transgenic lines were generated with the 35SCSB3 construct and some of them were studied in depth. All the transgenic lines generated had lost the characteristic morphological phenotype that can be attributed to the csb3-1 mutation and had recovered a natural outward appearance (see Figure 7A). Also, the transformants lost the constitutive expression of the P69CGUS gene (Figure 7A), as well as the constitutive expression of the SA related genes, that is PR-1 and GST6, characteristic of the non-transformed csb3-1 plants (Figure 7C). In addition, the expression of At5g60600 under the control of the 35S promoter in csb3-1 transgenic plants suppresses the resistance increase to Pst DC3000. In these transgenic plants resistance to Pst DC3000 returns to levels similar to those achieved by wild plants (Figure 7B). The complete complementation of all the different aspects of the csb3 phenotype confirms that At5g60600 is equivalent to CSB3.

### CSB3 expression is partially inhibited by Pst DC3000 infection

CSB3 expression as a response to *P. syringae* infection was analyzed in the leaves of wild plants at different time intervals after inoculation and in a context that generated a compatible interaction (that is, after Pst DC3000 inoculation) or an incompatible interaction (that is after Pst DC3000 (averRpm1) inoculation). The presence of CSB3 mRNA was studied using the RT-PCR analysis. These analyses revealed that Pst DC3000 infection with the avirulence gene - avrRpm1 gene - and that therefore incited HR in the inoculated tissue was followed by a marked decrease of the accumulation of CSB3 mRNA (Figure 7D). This reduction was already observed 24 hours after inoculation and was later maintained even 48 hours after inoculation.

In a concomitant manner and inversely correlated to this reduction, the SA inducible marker gene PR-1 was regulated by increment. Similar results were recorded, although these results had a less intense effect, in a compatible interaction such as that following inoculation with Pst DC3000 lacking avrRpm1 (Figure 7D). Also, CSB3 expression in csb3-1 plants also revealed a decrease of the stationary mRNA as compared with wild plants (Figure 7D), and therefore, regulation of the CSB3 by decrease after bacterial infection or in the mutant background is inversely correlated to the induced expression of PR-1.

### Phosmidomicine complements csb3-1 plants increased resistance to Pst DC3000

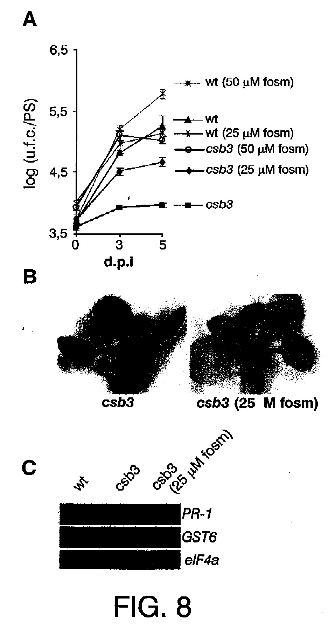

An increase in substrate (MEcPP) accumulation of the CSB3/HDS enzyme or of any of the intermediate metabolic products that precede MEcPP accumulation in the MEP pathway (Figure 6) could be responsible for the increased resistance of csb3-1 plants. Alternatively, a decrease of product synthesis (HMBPP) of the same enzyme could compromise the synthesis and accumulation of final products (IPP and DMAPP) (Figure 6) that in turn could compromise the later biosynthesis of the different isoprenoids that must be derived from the MEP pathway. This last possibility can also be responsible for the resistance increase of the csb3-1 plants. In order to study any of these possibilities the impact of an inhibitor in the MEP pathway for increased resistance of csb3-1 plants to Pst DC3000 was studied. To approach the question in this manner phosmidomicine [3-(N-formil-N-hydroxyamino)-propylphosphonic acid] was used as well as a DXR competitive inhibitor, the enzyme that controls the second stage involved in the MEP pathway (Figure 6) and reduces the levels of the final products obtained by this pathway (that is, IPP and DMAPP) (Schwender, J., Muller, C., Zeidler, J., and Lichtenthaler, H.K. (1999). Cloning and heterologous expression of a cDNA encoding 1-deoxy-D-xylulose- 5 phosphate reductoisomerase of Arabidopsis thaliana. FEBS Lett. 455, 140-144.; Laule, 0., Furholz, A., Chang, H., Zhu, T., Wang, X., Heifetz, P.B., Gruissem, W., and Lange, M. (2003). Crosstalk between cytosolic and plastidial pathways of isoprenoid biosynthesis in Arabidopsis thaliana. Proc. Natl. Acad. Sci. USA 100, 6866-6871). In order to separate this response from the response derived from an arrest of the chloroplast development, phosmidomicine treatments were applied in much smaller dosages and during smaller periods of times than those used experimentally by others (Laule et al., 2003). Wild seedlings and csb3-1 seedlings were sprayed once with a 25 µM or a 50 µM dilution of phosmidomicine 48 hours and 24 hours before inoculation with Pst DC3000 and the subsequent bacterial growth was recorded in inoculated plants at 0.3 and 5 d.p.i. Treated plants were also studied to determine the effect on the normal expression of the P69C-GUS gene. As shown in Figure 8A, treating the csb3-1 plants once with 25 µM phosmidomicine was sufficient to noticeably reduce resistance increase to Pst DC3000. This inhibiting effect was even more obvious after one 50 µM phosmidomicine treatment, which completely suppressed the resistance increase attributed to the csb3-1 mutation (Figure 8A). This last concentration of the inhibitor reduced the resistance response observed in the mutant plant to a level comparable to that reached by untreated wild plants. The 5 µM phosmidomicine treatments applied to the csb3-1 plants also provoked a reduction in the increased resistance of the csb3-1 plants that was only observed 5 days after inoculation with Pst DC3000, which indicates a dosage-related effect in the inhibiting action of phosmidomicine; a 50 µM concentration also showed an inhibiting effect on the normal resistance of wild plants to Pst DC3000, and made the plants more susceptible to this virulent pathogen (Figure8A). Greater dosages of this inhibitor were not tested, since they could have a collateral developmental and/or homeostatic effect in the chloroplast that could lead to undesired side effects.

This inhibiting effect of phosmidomicine on the increased resistance to bacterial growth of csb3-1 plants was also accompanied by a regulation by reduction of the constitutive expression of the P69G-GUS transgene (Figure 8B) and that of the PR-1 and GST6 marker genes (Figure 8C).

Consequently, it could be concluded that the increase in resistance of csb3-1 plants seems not to be so much related to the decrease of IPP and that of its isomer, DMAPP, but it is more the result of an excessive accumulation of the direct HDS/CSB3 substrate, MEcPP to be more specific, or of any of its immediate precursors. Also, the same compound seems to exert an effect on the control of at least the magnitude of resistance of wild plants against Pst DC3000 infection.

Therefore, a novel mutant of the CSB3 gene of *Arabidopsis thaliana* known as csb3-1 has been identified in the present invention. This novel mutant shows high levels of resistance to the biotrophic pathogens *P. syringae* and *H. parasitica,* while it retains normal susceptibility to necrotrophic pathogens *P. cucumerina* and *B. cinerea,* which is useful when it comes to obtain plants resistant to diseases caused by biotrophic pathogens. Phenotypic and genetic analyses indicate that csb3-1 is a partial loss of function recessive mutation that results in the activation of the plant's defenses. Consistently with the observed increase in resistance to biotrophic pathogens, csb3-1 plants show an increase in the SA accumulation in at rest conditions and an additional capacity to increase biosynthesis above the levels achieved by wild plants after exposure to Pst DC3000. This observation offers and explanation of why the csb3-1 plants also show constitutive activation of several marker genes related to defense (such as PR1, PR2 and GST6), that are finally controlled through the SA mediated signaling pathway (Durrant and Dong, 2004). The observation that SA is absolutely required for the resistance conferred by csb3-1 was particularly instructive in terms of understanding the role of CSB3 in this defence pathway, such as it is deduced by suppressing this phenotype in several of the double mutants that were generated. The levels of PR-1 genic transcript in stationary state, the P69C-GUS expression and the resistance increase to Pst DC3000 were suppressed in a rather noticeable manner in the csb3 pad4, csb3 sid2, csb3 eds5 and csb3 NahG double mutants when compared to the resistance increase observed in csb3-1 homozygotic plants.

All these observations indicate that the fact of maintaining the SA synthesis and accumulation processes intact is essential to increase resistance to disease in csb3-1 plants, and therefore, these studies indicate that the csb3-1 mediated control of the SA defensive response seems to act at some stage during the initial events that comprise the interaction between plant and pathogen. In addition, increase resistance of csb3-1 plants requires components that operate as transductors for the SA derived defense signal (such as NPR1), as shown by the doubly mutants csb3 nrp1 plants that behave as npr1 plants in terms of the lack of expression of the PR gene and hypersusceptibility to Pst DC3000 infection.

Also, the reversal of the resistance phenotype, from resistant to hypersusceptible, without suppression of the of the level of PR-1 genic transcript in stationary state in the csb3 dth9 double mutant, reveals also a need for an intact DTH9 dependent pathway after the 3' end of SA accumulation for the csb3-1 associated resistance. Consequently, it is required for both NPR1 and DTH9 pathways to be intact to regulate disease resistance in csb3-1 plants. Curiously, the resistance to disease phenotype of the csb3-1 plants is accompanied by the onset of an abnormal growth habit manifested as dwarfism that is accompanied by the presence of curly leaves in the basal rosette. This developmental alteration is also a characteristic shared by other *Arabidopsis* mutants amongst which the cpr mutants (constitutive expressors of PR Genes) are noteworthy (Durrant and Dong, 2004). Like csb3-1, cpr mutants are more resistant to biotrophic pathogen growth, they show constitutive expression of the PR-1 gene and are completely dependent of SA accumulation (Bowling et al., 1994), however, and unlike csb3-1, cpr mutants show independence from NPR1 in terms of increased resistance and also to express defenses (Clarke, J.D., Volko, S.M., Ledford, H., Ausubel, F.M., and Dong, X. (2000). Roles of salicylic acid, jasmonic acid, and ethylene in cpr-induced resistance in Arabidopsis. Plant Cell 12,2175-2190). Also, generation of doubly mutant csb3 cpr1 plants revealed that both mutations are additive, resulting in plants with an extremely aberrant phenotype that never reaches reproductive stage, consequently, csb3-1 and cpr1 mutations may contribute to their characteristic increase in SA accumulation and their respective phenotypes of increased resistance to disease through independent pathways.

The CSB3 gene was identified by using a positional cloning strategy. This gene complemented the csb3-1 mutation as shown, by reducing the PR genes expression, as well as by reversing the increased resistance in csb3-1 transgenic plants that express CSB3 (Figure 7A-C), confirming once again CSB3 epistasis to SA accumulation and the PR expression and increased resistance to Pst DC3000. In addition, analysis of the genic expression in wild plants revealed that CSB3 is expressed in a moderate manner in at rest conditions and the expression is regulated by reduction after Pst DC3000 infection. This regulation by reduction is more pronounced during incompatible interactions, such as that provoked during Pst DC3000 infection (avrRpm1). This regulation by reduction is inversely correlated, both in time and in intensity, with the observed induction of SA regulated genes (such as PR-1) during the same plant-pathogen interaction (Figure 7D). These observations suggests that regulation by reduction of the CSB3 transcript could be functionally involved in the accumulation of SA and the activation of defenses in plants after pathogen induction, and may coincide with CSB3, that acts as negative regulator of the SA dependent pathway leading to resistance to biotrophic pathogens in wild plants.

In the effort to identify suppressors of the csb3-1 mutant, known as scs for the purposes of the present invention, csb3-1 seeds were subjected to mutagenesis with EMS, and M2 plants were selected to find plants that had lost the characteristic dwarf phenotype and the csb3-1 resistance to pst DC3000. Four scs mutants were identified in which the increased resistance trait to this pathogen had reverted to a level comparable to that reached by wild plants (that is, in scs2 and scs11 plants. See Figure 4), or had been even additionally inhibited to make this plants hypersusceptible to the same pathogen (plants scs9 and scs11. See Figure 4). The identified scs mutants correspond to non-allelic recessive mutations that are extragenic for the csb3 locus and conferred the same phenotype even in the absence of the csb3-1 mutation. These observations provide additional evidence to the effect that CSB3 is an important regulator of the SA mediated resistance to diseases in *Arabidopsis.*

Likewise, these results suggest that the role of CSB3 in the resistance to disease response is under strict control such as it has been determined by identifying the SCS regulators. Additional characterization and cloning of the scs suppressor will help to understand in greater detail how this regulation mechanism works during the resistance to disease response.

The CSB3 gene codes 1-hydroxy-2-methyl-2-butenyl-4-diphosphate [HMBPP] synthase that catalyzes the formation of HMBPP from 2,4-cyclodiphosphate of 2-C-methyl-D-erytrythol [MEcPP] (Kollas et al., 2002). This enzymatic activity controls one of the terminal stages in the synthesis of isopentenyl diphosphate [IPP] and its isomer, dimehtylallyl diphosphate [DMAPP] through the 4-phosphate of 2-C-methyl-D-erithrytol (MEP) pathway independently from the mevalonate produced in the chloroplast of higher plants (Figure 6). The MEP pathway localized in the plastid, together with the cytosolic mevalonate (MVA) dependent pathway synthesize the isoprenoid precursors from which a plethora of different compounds, metabolites and plant hormones are originated, (Rohmer, 1999). It is not forecasted that the csb1-3 mutant will be a null mutant, since other null alleles in the CSB3 gene (csb3-2 and clb4/csb3-3) are mortal, and therefore, the partial loss of function attributed to substituting Gly by Asp as identified in the mutant csb3-1 protein may be explained when considering that the introduction of an aminoacid residue in place of a neutral residue inside or near the 4F3-4S motif, that is extremely conserved in these type of enzymes, may alter a correct conformation in this domain that, in turn, may destabilize to some degree the correct protein folding process. This coincides with prior observation of other mutations that occur in the same region of the *E. coli* homologous CGPE (Sauret-Güeto et al., 2003).

Because the loss of function associated to the csb3-1 mutation results in the activation of the SA pathway leading in turn to an observed increase in the resistance to disease, it has been deduced that the breakdown of a compound downstream of HDS/CSB3 (caused by enzymatic activity reduction of the csb3-1 enzyme) or, alternatively, the excessive accumulation of a compound previous to the action of the HDS/CSB3 enzyme (such as MecPP or any of its metabolic precursors before the 5' end) must have a role in the defensive response controlled by SA. This conjecture was tested by examining the effect of a MEP pathway inhibitor (phosmidomicine) on the resistance increase of csb3-1 plants as well as on the normal resistance of wild plants.

Phosmidomicine blocks the activity of DXR, the enzyme that controls the second stage of the MEP pathway (Figure 6), and consequently inhibits the pathway in a step prior to the action of HDS/CSB3 (Schwender et al., 1999; Laule et al., 2003). It was observed that the inhibiting effect of the phosmidomicine could complement completely the resistance increase phenotype associated to csb3-1, as well as the expression of the genes related to the defense function (Figure 8). In consequence, this suggests that activation of the SA pathway in the csb3-1 mutant is not due to a reduction of a compound that arises after the action of CSB3/HDS, that may compromise later enzymatic stages leading to isoprenoid synthesis, but on the contrary, it is the result of an excessive accumulation of the direct HDS/CSB3 substrate, specifically of MEcPP, or indirectly, of any of its immediate precursors. Also, phosmidomicine also affects the disease resistance response of wild plants against Pst DC3000, and 50 µM treatments of this inhibitor resulted in greater rates of bacterial growth (Figure 8A).

This finding reinforces, additionally, the insight that the same compound that accumulates in excess in the csb3-1 plants and regulates positively the increase of resistance to disease in a mutant plant is also important for a normal resistance to disease response in wild plants.

In summary, these results point in the direction of the existence of a chloroplastic signal/factor derived from one of the initial intermediate stages of the MEP pathway in the control of the pathogen activated SA pathway leading to disease resistance against biotrophic pathogens. This information supports the significance of the chloroplast in the mediation processes that affect the most critical aspects of the plant's defensive response. The future challenges are to determine which precise molecule or molecules are responsible for the control observed in the SA pathway, and how this action is controlled by the product of the SCS genes identified in the present invention.

### BRIEF DESCRIPTIONS OF THE FIGURES

**Figure 1****. Characterization of csb3 plants and comparison to wild plants.**
   (A) Comparison of the external aspect of csb3 plants (right) and wild plants (wt) (left). The plants were photographed when they were 3.5 weeks old.
   (B) Histochemical stain of GUS activity led by the P69C promoter in a 14 day old wild transgenic seedling (left) and csb3 seedlings (right).
   (C) Completely expanded rosette leaf from a wild transgenic plant (left) and csb3 plants (right) stained to show GUS activity.
   (D) Expression of PR-1, PR-2, GST6 AND PDF1.2 in wild plants and csb3.
   (E) Levels of free SA and total SA in wild plants and in csb3 plants. The wild plants (wt) and the csb3 plants were inoculated with 10 mM of Pst DC3000 or MgSO₄ (trial test) and the amount of free SA and SA glucoside (SAG) was measured 24 hours after inoculation. Each bar represents the average obtained from three replicate samples with the standard deviation calculated. Those same samples were tested for free
   SA and SA glucoside (SAG).
**Figure 2****. Resistance increase in csb3 plants against biotrophic but not necrotrophic pathogens.**
   (A) Resistance response of mutant Arabidopsis csb3 plants and wild Arabidopsis plants against virulent *Hyalopersonospora parasitica.* The leaves of 2 week old plants were stained with tryphan-lactophenol stain seven days after having been inoculated with a spray solution containing 10⁵ conidiospores per milliliter of water. The leaves were then observed under the microscope were they were seen to reveal the characteristic extensive hyfa growth. In csb3 plants the growth of this pathogen was remarkably inhibited.
   (B)To quantify the resistance to *H. parasitica,* the conidia production was counted 7 days after inoculation with the aid of a hemocytometer. The plants with the csb3 mutation showed to be extremely resistant to this pathogen.
   (C) Growth of *Pseudomonas syringae pv. tomato* DC3000 in wild plants and in csb3 plants. Two week old plants were inoculated by immersing them in a bacterial suspension. The bacterial titre was determined and measured as colony forming units (cfu) by fresh weight at days 0, 3 and 5 after infection in both wild and csb3 mutant plants. The data points shown represent the average ± the logarithms' standard deviation. The experiment was repeated three times with similar results. The plants with the csb3 mutation showed to be remarkably resistant to this pathogen.
   (D) Resistance response to wild plants and csb3 plants to *Plectosphaerella cucumerina.* Plants were inoculated by applying 6 µl drops of a spore containing suspension of *P. cucumerina* (5 x 10⁶ spores per ml) to fully expanded leaves. The symptoms of the disease were evaluated 4 days after inoculation by determining the average diameter of the lesion in 3 leaves of the 8 inoculated plants every time. The data points represent the average lesion size ± SD of the measurements taken. The plants carrying the csb3 mutation were as resistant to this pathogen as wild plants.
   (E) Leaves of wild plants and of csb3 plants 4 days after inoculation with a drop of 6 µl of *Botrytis cinerea* spores (2.5 x 10⁴ conidia per ml).
   (F) The size of the lesion generated by the *Botrytis cinerea* infection was measured 4 days after inoculation. The data points represent the average lesion size ± SD of the measures gathered for 30 lesions minimum. This experiment was repeated three times with similar results. (sic) (wt, wild). The csb3 mutant plants proved to be as resistant to this pathogen as wild plants.
**Figure 3****. Effect of SA related mutations on the resistance to disease response in csb3 plants**
   (A) Pst DC3000 growth in csb3 pad4, csb3 sid2, csb3 eds5, csb3 nahG, csb3 dth9, csb3 npr1 mutants compared to Pst DC3000 growth in simple mutants and wild plants. Bacterial growth was recorded in leaf samples picked up immediately after infection, 3 days after inoculation (dpi) and 5 dpi. Eight samples were taken per genotype for each time point. The experiment was repeated three times with similar results.
   (B) RT-PCR analysis of PR-1 and GST6 genic expression in SA related mutants, in csb3 double mutants and in wild plants. The lower gel shows the RT-PCR for the elF4α "housekeeping" gene used in the present invention as a load control.
   (C) Completely expanded rosette leaf from a wild plant (center), csb3 plants (left) and csb3 nahG plants (right) stained to determine GUS activity as directed by the promoter of gene P69C.
**Figure 4****. Characterization of the csb3 suppressors**
   (A) Upper frame: morphological phenotypes of the scs mutants that act as suppressors for the csb3 phenotype. All the plants were grown in a parallel formation on the ground and were photographed when they were 4 weeks old. Lower frame: suppression of the expression of the csb3 induced P69C-GUS indicator gene in leaves from scs suppressor mutant plants.
   (B-C) Increase of susceptibility to disease caused by Pst DC3000 in the different scs mutants. Bacterial growth was recorded at days 0, 3 and 5 post inoculation (dpi). The experiment was repeated twice obtaining similar results.
**Figure 5****. Positional cloning and CSB3 structure**
   (A) 0.8 Mb region in chromosome 5 having overlapped BAC flanked by the SSLP, Ngal29 and MBK-5 markers used to detect recombination in 1496 chromosomes.
   (B) Location of CSB3 in the sequenced region corresponding to the MUF9 BAC clone. CSB3 was located between two SSLP markers that encompass a 66kb region.
   (C)Structure of a CSB3 exon/intron. The thick lines indicate coding regions. The positions of the csb3-1, csb3-2 and clb4/csb3-3 mutations are shown in the upper part of each corresponding exon. The insert represents the nucleotide exchange found in the csb3-1 mutant in its influence on the protein sequence. The mutant allele is shown under the wild plant sequence. Capital letters show the nucleotide sequences found in the half section of exon 19. Transition from G to A caused by the mutagen is shown in bold type. The aminoacid sequences deduced are shown as an individual letter code under each nucleotide triplet and the bold type letters show aminoacid changes (G to D) in protein sequences.
   (D) Deduced aminoacid sequence belonging to the hydroxymethylbutenyl-4-diphosphate synthase (HDS, or also known as GcpE in microorganisms) associated to the plastid and codified by CSB3. The sequence signal forecasted for the plastid transport (ChloroP program) is shown underlined in the N-terminal end. The homologous regions between HDS homologous of plants and the GcpE homologous from microorganisms are shown in italics. The protein domain found between these two conserved domains is specific to the plant, since it is not present in the GcpE enzyme from the microorganisms. In the C-terminal end all the cystein residues preserved that are extremely conserved in all the HDS and GcpE enzymes are marked by an asterisk. The conserved residue that comprises the signature where the cluster [4Fe-4S] (Sauret-Güeto et al., 2003) is located is shown underlined. The glycine residue converted to aspartic residue in the csb3-1 mutant is shown in bold type in position 695 and is close to the conserved sequence that participates in the coordination of [4Fe-4S].
**Figure 6****. Diagram of the metabolic pathway of 4-methyl phosphate-D-erythrytol (MEP)**
   The abbreviations of the enzymes that catalyze each stage of the pathway are shown on the left side of the figure in bold type as shown: 1-desoxy-D-xyllulose-5-synthase phosphate (DXS); 2C-methyl-D-erythrytol-4-synthase phosphate (DXR); 4-diphosphocitidil-2C-methyl-D-erythrytol-4-phosphate synthase (CMS); 4-diphosphocitidil-2C-methyl-D-erythrytol kinase (CMK): 2C-methyl-D-erythrytol-2,4-diphosphate synthase (MCS); 2C-methyl-D-erythrytol-2,4-cyclodiphosphate reductase (HDS); 1-hydroxy-2-methyl-butenyl reductase-4-diphosphate (HDR); isopenthenyl-diphosphate isomerase (IDI). The names of some of the mutants found are shown on the right side of the diagram. The position of the csb3 mutation is shown. The position at which it is known that phosmidomicine inhibits the pathway is also shown.
**Figure 7****. Complementation of csb3-1 plants and regulation by reduction of the CSB3 genic expression after Pst DC3000 infection**
   (A-C) Phenotypic and molecular characterization of csb3-1 plants transformed in a stable manner with a 35S-CSB3 genic construct. (A) morphological aspect of non-transformed csb3-1 plants compared to two transgenic lines. Please observe the reversion towards a wild phenotype in the transgenic lines. The histochemical stain of the GUS activity led by the P69C promoter in fully expanded rosette leaves obtained from non-transformed csb3-1 plants and from the plants of the two transgenic lines is shown in the same frame.
   (B) Resistance response to Pst DC3000 of two independent csb3 transgenic lines (line 1.1 and line 5.6) transformed in a stable manner with a 35S-CSB3 genic construct and comparison of the resistance response observed in the wild type (wt) and in the csb3-1 mutant plant. Please observe that the transgenic lines have lost the increased resistance associated to the csb3-1 mutation. Bacterial growth was recorded on days 0, 3 and 5 after inoculation (dpi). The experiment was repeated twice with the same results.
   (C) RT-PCR analysis of the PR-1 and GST6 genic expression in csb3-1 plants and in wild plants and in the transgenic lines (lines 1.1 and 5.6) expressing the 35S-CSB3 genic construct. Pleas observe that the transgenic plants no longer express in a constitutive manner the SA related marker genes. The lower gel shows the RT-PCR results for the elF4α housekeeping gene used in the present invention as load control.
   (D) RT-PCR analysis of the CSB3 and PR-1 genic expression in wild plants after Pst DC3000 infection that carry or do not carry the avrRpm1 avirulence gene. The numbers indicate hours after inoculation. The expression level of these two genes was also analyzed in healthy csb3-1 plants and it is shown on the left as comparison. The lower gel shows the RT-PCR for the elF4α housekeeping gene used in the present invention as load control. The experiment was repeated twice with the same results.
**Figure 8****. Pharmacological complementation with phosmidomicine of the resistance increase in csb3-1 plants.**
   (A) Wild seedlings (wt) and csb3-1 seedlings of *Arabidopsis* were sprayed once at 48 hours and 24 hours before exposing them to Pst DC3000 with a buffer solution complemented with 25 µM or 50 µM dilution (+ phosm.) the controls were treated in a similar manner with buffer solution alone. The plants were exposed to Pst DC3000 and the pathogen's growth was recorded at days 0, 3 and 5 after inoculation (dpi).
   (B) The effect of the phosmidomicine treatment (25 µM, treated as described in point (A) on the constitutive expression of P69C-GUS in csb3-1 seedlings (right) as compared with csb3-1 seedlings treated with the buffer solution alone (left).
   (C)The effect of the phosmidomicine treatment (25 µM, treated as described in point (A) on the constitutive expression of SA regulated marker genes in csb3-1 seedlings of *Arabidopsis* as compared with csb3-1 seedlings treated with the buffer solution alone.

### DETAILED DESCRIPTION OF ONE MODE OF MATERIALIZATION

### Plants, growth conditions and treatments.

*Arabidopsis thaliana* plants were grown on the ground or in plates containing the Murashige and Skoog growing media (MS) such as has been previously described (Mayda et al., 2000). The csb3-1 mutant was isolated in one selection to determine which express in a constitutive manner the P69C-GUS indicator gene in Columbia transgenic plants (Col-0) subject to mutagenesis with ethyl methanesulfonate (EMS) as has been previously described for other mutants (Mayda et al., 2000).

The mutant line csb3-1 used in these experiments had been subjected to backcrossing at least four times with the original wild line. Plants were made to grow in a growth chamber (19 - 23°C, fluorescent light at 100 µEm-₂ seg-₁) in a cycle of 10 hours of light to 14 hours of darkness. The leaves used, unless otherwise indicated, were fully expanded leaves of 4 week old plants for all the experiments. The stain to establish GUS activity was done as previously described (Mayda et al., 2000).

### Pathogenic infection

*Pseudomonas syringae pv. tomato* DC3000 was grown and was then prepared to be inoculated as previously described (Mayda et al., 2000). The plants were inoculated by immersion according to the method described by Tornero, P. and Dangl, J. L. (2001). A high throughput method for quantifying growth of phytopathogenic bacteria in Arabidopsis thaliana. Plant J. 28: (4): 475-481.The density of the bacteria population was determined by culture in dilution plates series on B King media complemented with rifampicine (50 µg/ml) at 28°C and counting the colony forming units at different times as previously described (Mayda et al., 2000). The data was recorded as means and standard deviations of the logarithm (cfu/g of fresh weight) calculated from six to eight replicates. Trial tests to measure the resistance against *Hyaloperonospora parasitica* were carried out in three week old plants by spraying them with a suspension of 10⁵ conidiospores per ml₋₁ of tap water as has been previously described (Mayda et al., 2000). On the seventh day the spore density on the plantlets was assessed (seven pots per treatment, each pot treated separately) using a hemocytometer. Alternatively, leaf samples were stained with tripano-lactophenol blue in different days after inoculation and the leaves thus stained were examined under the microscope as has been already described (Mayda et al., 2000).

To measure resistance to *Plectosphaerella* and *Botrytis* three week old seedlings were transplanted to individual pots and were grown at 22°C (day temperature) and at 18°C (night temperature) in light cycles of 12 hours of light every 24 hours. When the plants reached 6 weeks of age they were inoculated by application of 6 µl drops of a suspension containing spores of *Plectosphaerella cucumerina* (5 x 10⁶ spores ml-₁) or of *Botrytis cinerea* (2.5 x 10⁴ conidia ml-₁) to 3 fully expanded leaves per plant.

*P. cucumerina* was isolated from *Arabidopsis* with a wild infection (Landsberg *erecta* registry) and it was grown on 19.5 g/L of potato dextrose agar (Difco, Detroit, MI) at ambient temperature during 2 weeks before collecting the spores and suspending them in MgSO₄ 10 mM *B. cinerea* (BMM1 strain isolated from *Pelargonium zonale*) and then grow them on 19.5 g/L of potato dextrose agar (Difco, Detroit, MI) at 20°C during 10 days. The conidia were collected and suspended in sterile PDS (12 g I₋₁; Difco). The plants were kept at 100% HR and the symptoms of the disease were evaluated 4 to 10 days after inoculation determining the average diameter of the lesion in 3 leaves of 5 plants each time.

### Genetic analysis

Crosses were done by cutting non-open shoots and using the pistils as pollen recipient. Backcrossing with the original transgenic line were done using P69C-GUS plants as pollen donors. Reciprocal crosses were also performed. F1 and F2 plants were grown in MS plates and were analyzed to determine GUS activity. Phenotype segregation was analyzed in the F2 generation to determine the goodness of fit with the chi-square test

### Map-based cloning

A csb3 plant was crossed (in the Columbia background) with Landsberg *erecta* and homozygotic csb3 mutants were selected for mapping amongst the segregating F2 offspring. Recombinant seedlings were identified using the single sequence length polymorphism markers (SSLP) according to the protocol described by Bell and Ecker (1994) and with new markers as reported in the web site of the *Arabidopsis* database (www.arabidopsis.org).

### Double mutants generation

The alleles of the mutants used throughout this study were npr1-1 (Cao et al. 1997), pad4-1, sid2-1 (Wildermuth et al., 2001), eds5 (Nawrath et al., 2002) and dth9 (Mayda et al., 2000). The transgenic plant has been described (in the Columbia ecotype) that expresses the NahG bacterial gene. The csb3 npr1, csb3 pad4, csb3 sid2, csb3 eds5, csb3 dth9 and csb3 NahG double mutants were generated using csb3 as pollen receptor. The homozygotic characteristic of the loci was confirmed using a molecular marker for each of the alleles in the segregating populations. For the dth9 containing double mutant, hypersusceptibility to Pst DC3000 was the criteria used to assess the homozygotic trait. All double mutants were confirmed in generation F3.

### cDNA cloning and inverse complementation

The genomic sequence was used as base for cDNA cloning of CSB3. Poli (A+) RNA of wild plants was isolated and reverse transcription was done using (dT) oligo primers as has been described (Mayda, E., Tornero, P., Conejero, V., and Vera, P. (1999)). A tomato homeobox gene (HD-Zip) is involved in limiting the spread of programmed cell death. Plant J. 20, 591-600). This cDNA was amplified by PC using gene specific primers designed to include the region before the 5' end of the initiation codon and part of region 3' that follows the CSB3 gene termination code. The direct and inverse primers' sequences for CSB3 used were as follows: (5'-CTTCCTCTGGATCCTTCTCTTCTC-3') and 5'-GGACACTAGTTCAAAATGATGATG-3') respectively. The cDNA was cloned in the binary pBI121 vector (Clontech) under the control of the 35S constitutive promoter of CaMV. From there the construct was transferred to Pcambia1300 which resulted in pCAMBIA35SCSB3, that was in turn transferred to *Agrobacterium,* and was then used to transform the csb3-1 plants by means of the floral immersion method (Becthold, N., Ellis, J. and Pelletier, G. (1993). In planta Agrobacterium mediated gene transfer by infiltration of adult Arabidopsis thaliana plants. C.R. Acad. Sci. Paris Life Sci. 316, 1194-1199).

### SA determination

The free and the conjugated SA content in leaves was determined in methanolic extracts by HPLC analysis as has been previously described (Mayda et al., 1999). **Expression analysis**

To analyze the level of genic expression by inverse transcriptase mediated PCR, the total RNA samples from leaf tissue were prepared using the Totally RNA kit made by Ambion (Austin, TX). Inverse transcription was done using the RT kit for PCR made by Clontech (Palo Alto, CA). The sets of oligonucleotidic primers (50 pmol each) used to amplify CSB3 were as follows: CSB3PCR1 (5'-GGAGGCCTTCTTGTGGATGG-3') / CSB3PCR2 (5' GCTGACCCAACGACCATGTTC-3'). The primers used to amplify GST6 were the following: GST6PCR1 (5'-ATGGCAGGAATCAAAGTTTCGGTC3') / GST6PCR2 (5'-GAGATTCACTTAAAGAACCTTCTG-3'). The primers used to amplify PR1 were: PR1 PCR1 (5'- ATGAATTTTACTGGCTATTC-3') / PR1 PCR2 (5'- AACCCACATGTTCACGGCGGA-3').

## Claims

1. A mutant allele of the CSB3 gene of *Arabidopsis thaliana* called csb3-1, **characterized by** its SEC.ID.NO.1 sequence in which it presents a substitution of a Glycine residue by an Aspartic Acid residue in position 695.

2. Mutant csb3-1 according to claim 1, **characterized in that** it confers plants resistance against diseases produced by biotrophic pathogens.

3. Mutant csb3-1 according to the previous claims, **characterized in that** it confers constitutive expression to genes PR-1, PR-2 and GST6, that is completely dependent on the accumulation of salicylic acid.

4. Use of the mutant allele csb3-1 in crop species of agricultural interest to obtain plants resistant to diseases caused by biotrophic pathogens.

5. Use of CSB3 as negative regulator of the salicylic acid mediated disease resistance response against biotrophic pathogens.

6. Transgenic plants **characterized in that** they incorporate the CSB3 gene in which the incorporated gene has been genetically manipulated in such a manner that its genic expression or its functionality has been repressed or altered, conferring them a greater resistance to biotrophic pathogens.

7. Mutant csb3-1 plants **characterized in that** the resistance increase they show against biotrophic pathogens is determined by the loss of function of the CSB3 gene, and therefore of the 1-hydroxy-2-methyl-2-butenyl-4-diphosphate synthase enzyme that said gene codes and that must result in an over-accumulation of its immediate substrate, that is, 2,4-cyclodiphosphate of 2-C-methyl-D-erythrytol or of some of the metabolic intermediates that precede it according to the MEP pathway.


SEQUENCE LIST

<110>  Consejo Superior de Investigaciones Científicas
       Universidad Politécnica de Valencia

<120>  Isolation and characterization of the CSB3-1 mutant of
       Arabidopsis thaliana and use thereof as a regulator of
       resistance in plants to diseases caused by biotrophic pathogens.

<130>  P-INCI-N-05-0089

<160>  2

<170>  PatentIn version 3.3

<210>  1
<211>  740
<212>  PRT
<213>  Arabidopsis thaliana

<400>  1

Met Ala Thr Gly Val Leu Pro Ala Pro Val Ser Gly Ile Lys Ile Pro
1               5                   10                  15

Asp Ser Lys Val Gly Phe Gly Lys Ser Met Asn Leu Val Arg Ile Cys
            20                  25                  30

Asp Val Arg Ser Leu Arg Ser Ala Arg Arg Arg Val Ser Val Ile Arg
        35                  40                  45

Asn Ser Asn Gln Gly Ser Asp Leu Ala Glu Leu Gln Pro Ala Ser Glu
    50                  55                  60

Gly Ser Pro Leu Leu Val Pro Arg Gln Lys Tyr Cys Glu Ser Leu His
65                  70                  75                  80

Lys Thr Val Arg Arg Lys Thr Arg Thr Val Met Val Gly Asn Val Ala
                85                  90                  95

Leu Gly Ser Glu His Pro Ile Arg Ile Gln Thr Met Thr Thr Ser Asp
            100                 105                 110

Thr Lys Asp Ile Thr Gly Thr Val Asp Glu Val Met Arg Ile Ala Asp
        115                 120                 125

Lys Gly Ala Asp Ile Val Arg Ile Thr Val Gln Gly Lys Lys Glu Ala
    130                 135                 140

Asp Ala Cys Phe Glu Ile Lys Asp Lys Leu Val Gln Leu Asn Tyr Asn

145                 150                 155                 160

Ile Pro Leu Val Ala Asp Ile His Phe Ala Pro Thr Val Ala Leu Arg
                165                 170                 175

Val Ala Glu Cys Phe Asp Lys Ile Arg Val Asn Pro Gly Asn Phe Ala
            180                 185                 190

Asp Arg Arg Ala Gln Phe Glu Thr Ile Asp Tyr Thr Glu Asp Glu Tyr
        195                 200                 205

Gln Lys Glu Leu Gln His Ile Glu Gln Val Phe Thr Pro Leu Val Glu
    210                 215                 220

Lys Cys Lys Lys Tyr Gly Arg Ala Met Arg Ile Gly Thr Asn His Gly
225                 230                 235                 240

Ser Leu Ser Asp Arg Ile Met Ser Tyr Tyr Gly Asp Ser Pro Arg Gly
                245                 250                 255

Met Val Glu Ser Ala Phe Glu Phe Ala Arg Ile Cys Arg Lys Leu Asp
            260                 265                 270

Tyr His Asn Phe Val Phe Ser Met Lys Ala Ser Asn Pro Val Ile Met
        275                 280                 285

Val Gln Ala Tyr Arg Leu Leu Val Ala Glu Met Tyr Val His Gly Trp
    290                 295                 300

Asp Tyr Pro Leu His Leu Gly Val Thr Glu Ala Gly Glu Gly Glu Asp
305                 310                 315                 320

Gly Arg Met Lys Ser Ala Ile Gly Ile Gly Thr Leu Leu Gln Asp Gly
                325                 330                 335

Leu Gly Asp Thr Ile Arg Val Ser Leu Thr Glu Pro Pro Glu Glu Glu
            340                 345                 350

Ile Asp Pro Cys Arg Arg Leu Ala Asn Leu Gly Thr Lys Ala Ala Lys
        355                 360                 365

Leu Gln Gln Gly Ala Pro Phe Glu Glu Lys His Arg His Tyr Phe Asp
    370                 375                 380

Phe Gln Arg Arg Thr Gly Asp Leu Pro Val Gln Lys Glu Gly Glu Glu
385                 390                 395                 400

Val Asp Tyr Arg Asn Val Leu His Arg Asp Gly Ser Val Leu Met Ser
                405                 410                 415

Ile Ser Leu Asp Gln Leu Lys Ala Pro Glu Leu Leu Tyr Arg Ser Leu
            420                 425                 430

Ala Thr Lys Leu Val Val Gly Met Pro Phe Lys Asp Leu Ala Thr Val
        435                 440                 445

Asp Ser Ile Leu Leu Arg Glu Leu Pro Pro Val Asp Asp Gln Val Ala
    450                 455                 460

Arg Leu Ala Leu Lys Arg Leu Ile Asp Val Ser Met Gly Val Ile Ala
465                 470                 475                 480

Pro Leu Ser Glu Gln Leu Thr Lys Pro Leu Pro Asn Ala Met Val Leu
                485                 490                 495

Val Asn Leu Lys Glu Leu Ser Gly Gly Ala Tyr Lys Leu Leu Pro Glu
            500                 505                 510

Gly Thr Arg Leu Val Val Ser Leu Arg Gly Asp Glu Pro Tyr Glu Glu
        515                 520                 525

Leu Glu Ile Leu Lys Asn Ile Asp Ala Thr Met Ile Leu His Asp Val
    530                 535                 540

Pro Phe Thr Glu Asp Lys Val Ser Arg Val His Ala Ala Arg Arg Leu
545                 550                 555                 560

Phe Glu Phe Leu Ser Glu Asn Ser Val Asn Phe Pro Val Ile His His
                565                 570                 575

Ile Asn Phe Pro Thr Gly Ile His Arg Asp Glu Leu Val Ile His Ala
            580                 585                 590

Gly Thr Tyr Ala Gly Gly Leu Leu Val Asp Gly Leu Gly Asp Gly Val
        595                 600                 605

Met Leu Glu Ala Pro Asp Gln Asp Phe Asp Phe Leu Arg Asn Thr Ser
    610                 615                 620

Phe Asn Leu Leu Gln Gly Cys Arg Met Arg Asn Thr Lys Thr Glu Tyr
625                 630                 635                 640

Val Ser Cys Pro Ser Cys Gly Arg Thr Leu Phe Asp Leu Gln Glu Ile
                645                 650                 655

Ser Ala Glu Ile Arg Glu Lys Thr Ser His Leu Pro Gly Val Ser Ile
            660                 665                 670

Ala Ile Met Gly Cys Ile Val Asn Gly Pro Gly Glu Met Ala Asp Ala
        675                 680                 685

Asp Phe Gly Tyr Val Gly Asp Ser Pro Gly Lys Ile Asp Leu Tyr Val
    690                 695                 700

Gly Lys Thr Val Val Lys Arg Gly Ile Ala Met Thr Glu Ala Thr Asp
705                 710                 715                 720

Ala Leu Ile Gly Leu Ile Lys Glu His Gly Arg Trp Val Asp Pro Pro
                725                 730                 735

Val Ala Asp Glu
            740

<210>  2
<211>  740
<212>  PRT
<213>  Arabidopsis thaliana

<400>  2

Met Ala Thr Gly Val Leu Pro Ala Pro Val Ser Gly Ile Lys Ile Pro
1               5                   10                  15

Asp Ser Lys Val Gly Phe Gly Lys Ser Met Asn Leu Val Arg Ile Cys
            20                  25                  30

Asp Val Arg Ser Leu Arg Ser Ala Arg Arg Arg Val Ser Val Ile Arg
        35                  40                  45

Asn Ser Asn Gln Gly Ser Asp Leu Ala Glu Leu Gln Pro Ala Ser Glu
    50                  55                  60

Gly Ser Pro Leu Leu Val Pro Arg Gln Lys Tyr Cys Glu Ser Leu His
65                  70                  75                  80

Lys Thr Val Arg Arg Lys Thr Arg Thr Val Met Val Gly Asn Val Ala
                85                  90                  95

Leu Gly Ser Glu His Pro Ile Arg Ile Gln Thr Met Thr Thr Ser Asp
            100                 105                 110

Thr Lys Asp Ile Thr Gly Thr Val Asp Glu Val Met Arg Ile Ala Asp
        115                 120                 125

Lys Gly Ala Asp Ile Val Arg Ile Thr Val Gln Gly Lys Lys Glu Ala
    130                 135                 140

Asp Ala Cys Phe Glu Ile Lys Asp Lys Leu Val Gln Leu Asn Tyr Asn
145                 150                 155                 160

Ile Pro Leu Val Ala Asp Ile His Phe Ala Pro Thr Val Ala Leu Arg
                165                 170                 175

Val Ala Glu Cys Phe Asp Lys Ile Arg Val Asn Pro Gly Asn Phe Ala
            180                 185                 190

Asp Arg Arg Ala Gln Phe Glu Thr Ile Asp Tyr Thr Glu Asp Glu Tyr
        195                 200                 205

Gln Lys Glu Leu Gln His Ile Glu Gln Val Phe Thr Pro Leu Val Glu
    210                 215                 220

Lys Cys Lys Lys Tyr Gly Arg Ala Met Arg Ile Gly Thr Asn His Gly
225                 230                 235                 240

Ser Leu Ser Asp Arg Ile Met Ser Tyr Tyr Gly Asp Ser Pro Arg Gly
                245                 250                 255

Met Val Glu Ser Ala Phe Glu Phe Ala Arg Ile Cys Arg Lys Leu Asp
            260                 265                 270

Tyr His Asn Phe Val Phe Ser Met Lys Ala Ser Asn Pro Val Ile Met
        275                 280                 285

Val Gln Ala Tyr Arg Leu Leu Val Ala Glu Met Tyr Val His Gly Trp
    290                 295                 300

Asp Tyr Pro Leu His Leu Gly Val Thr Glu Ala Gly Glu Gly Glu Asp
305                 310                 315                 320

Gly Arg Met Lys Ser Ala Ile Gly Ile Gly Thr Leu Leu Gln Asp Gly
                325                 330                 335

Leu Gly Asp Thr Ile Arg Val Ser Leu Thr Glu Pro Pro Glu Glu Glu
            340                 345                 350

Ile Asp Pro Cys Arg Arg Leu Ala Asn Leu Gly Thr Lys Ala Ala Lys
        355                 360                 365

Leu Gln Gln Gly Ala Pro Phe Glu Glu Lys His Arg His Tyr Phe Asp
    370                 375                 380

Phe Gln Arg Arg Thr Gly Asp Leu Pro Val Gln Lys Glu Gly Glu Glu
385                 390                 395                 400

Val Asp Tyr Arg Asn Val Leu His Arg Asp Gly Ser Val Leu Met Ser
                405                 410                 415

Ile Ser Leu Asp Gln Leu Lys Ala Pro Glu Leu Leu Tyr Arg Ser Leu
            420                 425                 430

Ala Thr Lys Leu Val Val Gly Met Pro Phe Lys Asp Leu Ala Thr Val
        435                 440                 445

Asp Ser Ile Leu Leu Arg Glu Leu Pro Pro Val Asp Asp Gln Val Ala
    450                 455                 460

Arg Leu Ala Leu Lys Arg Leu Ile Asp Val Ser Met Gly Val Ile Ala
465                 470                 475                 480

Pro Leu Ser Glu Gln Leu Thr Lys Pro Leu Pro Asn Ala Met Val Leu
                485                 490                 495

Val Asn Leu Lys Glu Leu Ser Gly Gly Ala Tyr Lys Leu Leu Pro Glu
            500                 505                 510

Gly Thr Arg Leu Val Val Ser Leu Arg Gly Asp Glu Pro Tyr Glu Glu
        515                 520                 525

Leu Glu Ile Leu Lys Asn Ile Asp Ala Thr Met Ile Leu His Asp Val
    530                 535                 540

Pro Phe Thr Glu Asp Lys Val Ser Arg Val His Ala Ala Arg Arg Leu
545                 550                 555                 560

Phe Glu Phe Leu Ser Glu Asn Ser Val Asn Phe Pro Val Ile His His
                565                 570                 575

Ile Asn Phe Pro Thr Gly Ile His Arg Asp Glu Leu Val Ile His Ala
            580                 585                 590

Gly Thr Tyr Ala Gly Gly Leu Leu Val Asp Gly Leu Gly Asp Gly Val
        595                 600                 605

Met Leu Glu Ala Pro Asp Gln Asp Phe Asp Phe Leu Arg Asn Thr Ser
    610                 615                 620

Phe Asn Leu Leu Gln Gly Cys Arg Met Arg Asn Thr Lys Thr Glu Tyr
625                 630                 635                 640

Val Ser Cys Pro Ser Cys Gly Arg Thr Leu Phe Asp Leu Gln Glu Ile
                645                 650                 655

Ser Ala Glu Ile Arg Glu Lys Thr Ser His Leu Pro Gly Val Ser Ile
            660                 665                 670

Ala Ile Met Gly Cys Ile Val Asn Gly Pro Gly Glu Met Ala Asp Ala
        675                 680                 685

Asp Phe Gly Tyr Val Gly Gly Ser Pro Gly Lys Ile Asp Leu Tyr Val
    690                 695                 700

Gly Lys Thr Val Val Lys Arg Gly Ile Ala Met Thr Glu Ala Thr Asp
705                 710                 715                 720

Ala Leu Ile Gly Leu Ile Lys Glu His Gly Arg Trp Val Asp Pro Pro
                725                 730                 735

Val Ala Asp Glu
            740